(19) Europäisches Patentamt
European Patent Office
Office européen des brevets

(11) Veröffentlichungsnummer: **0 048 891**
**B1**

(12) **EUROPÄISCHE PATENTSCHRIFT**

(45) Veröffentlichungstag der Patentschrift: **28.09.83**

(51) Int. Cl.³: **C 07 C 69/06**, C 07 C 67/36

(21) Anmeldenummer: **81107281.8**

(22) Anmeldetag: **16.09.81**

(54) **Verfahren zur Herstellung von Methylformiat.**

(30) Priorität: **01.10.80 DE 3037089**

(43) Veröffentlichungstag der Anmeldung: **07.04.82 Patentblatt 82/14**

(45) Bekanntmachung des Hinweises auf die Patenterteilung: **28.09.83 Patentblatt 83/39**

(84) Benannte Vertragsstaaten: **BE DE FR GB IT NL**

(56) Entgegenhaltungen:
**DE-A-1 147 214**
**DE-A-2 710 726**

(73) Patentinhaber: **BASF Aktiengesellschaft, Carl-Bosch-Strasse 38, D-6700 Ludwigshafen (DE)**

(72) Erfinder: **Mueller, Franz-Josef, Dr., Mueller-Thurgau-Weg 1, D-6706 Wachenheim (DE)**
Erfinder: **Steiner, Wolfgang, Dr., Hauptstrasse 38, D-6701 Friedelsheim (DE)**
Erfinder: **Ross, Karl-Heinz, Dr., Sudetenstrasse 8, D-6704 Mutterstadt (DE)**
Erfinder: **Kratzer, Otto, Dr., An der Tuchbleiche 7, D-6712 Bobenheim-Roxheim (DE)**

**EP 0 048 891 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Verfahren zur Herstellung von Methylformiat

Die vorliegende Erfindung betrifft ein verbessertes Verfahren zur Herstellung von Methylformiat durch Umsetzung von Methanol und Kohlenmonoxid in Gegenwart eines Alkali- oder Erdalkalialkoholates bei 30 bis 150°C und 50 bis 300 bar.

Dieses Verfahren, sieht man von der erfindungsgemäßen Verbesserung ab, ist allgemein bekannt, z. B. aus Ullmann, »Enzyklopädie der Technischen Chemie«, 3. Auflage (1953), Band 3, Seite 450-451.

Die technische Durchführung dieser Synthese ist jedoch mit verfahrenstechnischen Schwierigkeiten verbunden, die darauf beruhen, daß die Löslichkeit der Alkoholate im Reaktionsgemisch mit steigendem Methanolumsatz abnimmt, so daß es zu Salzabscheidungen und Verkrustungen in den Apparaturen kommt. Hierdurch wird nicht nur die Synthese des Methylformiates gestört, sondern auch die destillative Aufarbeitung des Reaktionsgemisches.

Dieses Problem ist Gegenstand von mehreren Patentveröffentlichungen, darunter der DE-B 1 147 214 und der DE-A-2 710 726 sowie dem dort erwähnten Stand der Technik, wurde bisher aber noch nicht befriedigend gelöst.

Nach dem Verfahren der DE-B-1 147 214 werden die Ablagerungen während der Carbonylierung des Methanols dadurch vermieden, daß man Methanol und Kohlenmonoxid in einem senkrecht stehenden Reaktor im Gegenstrom zueinander führt, daß man das Kohlenmonoxid dem Reaktor an mindestens zwei Stellen verschiedener Höhe zuführt und daß man das Mengenverhältnis dieser CO-Teilströme von Zeit zu Zeit kurzfristig ändert. Daß eine solche unstetige Arbeitsweise verfahrenstechnisch nachteilig ist, liegt indes auf der Hand.

Ebenfalls besonderer Vorkehrungen bedarf es beim Verfahren der DE-A-2 710 726, demzufolge man die Niederschläge durch intensive Dispersion des Kohlenmonoxids in der Flüssigphase zu vermeiden trachtet. Hierbei wird das flüssige Reaktionsgemisch über einen Außenkreislauf vom Reaktor abgezogen und danach in diesen wieder unter Ansaugen des Kohlenmonoxids eingepreßt.

Abgesehen davon, daß auch diese Arbeitsweise technisch aufwendig ist, trägt sie sowenig wie das Verfahren der DE-B-1 147 214 dazu bei, die Bildung der Niederschläge bei der Aufarbeitung der Reaktionsgemische zu verhindern.

Der Erfindung lag daher die Aufgabe zugrunde, den genannten Nachteilen abzuhelfen, indem die Salzabscheidungen weitgehend unterdrückt werden.

Demgemäß wurde ein Verfahren zur Herstellung von Methylformiat durch Umsetzung von Methanol und Kohlenmonoxid in Gegenwart eines Alkali- oder Erdalkalialkoholates bei 30—150°C und 50—300 bar gefunden, welches dadurch gekennzeichnet ist, daß man hierbei einen nichtionischen Komplexbildner zur Bindung der Alkali- bzw. Erdalkalimetallkationen mitverwendet.

Geeignete Komplexbildner sind vornehmlich cyclische und acyclische Verbindungen, für welche das Polyethylenglykol-Strukturelement

$$-(-O-CH_2-CH_2-)_n- \qquad n=2-12$$

für die Komplexbildungsfähigkeit charakteristisch ist. In diesen Verbindungen können die Sauerstoffatome der Struktureinheiten auch durch S oder vor allem —NH— ersetzt sein.

Beispiele für handelsübliche Verbindungen dieser Art sind

1,4,7,10,13-Pentaoxa[13]orthocyclophan (Benzo-15-C-5)
1,4,7,14,17,20-Hexaoxa[7,7]orthocyclophan (Dibenzo-18-C-6)
1,4,7,10,17,20,23,26-Octaoxa[10,10]orthocyclophan (Dibenzo-24-C-8)
2,5,8,15,18,21-Hexaoxatricyclo[20,4,0,$0^{9,14}$]hexacosan (Dicyclohexyl-18-C-6)
1,4,7,10-Tetraoxacyclododecan (12-C-4)
1,4,7,10,13-Pentaoxacyclopentadecan (15-C-5)
1,4,7,10,13,16-Hexaoxacyclooctadecan (18-C-6)
4,7,13,18-Tetraoxa-1,10-diazabicyclo-[8,5,5]-eicosan
4,7,13,16,21-Pentaoxa-1,10-diazabicyclo-[8,8,6]-tricosan

Außerdem eignen sich u. a.

Polyethylenglycoldialkylether (Alkyl z. B. $C_1-C_4$)
Triethanolamin
Tetraalkylethylendiamine (Alkyl z. B. $C_1-C_4$)

Näheres über diese und weitere geeignete Komplexbildner ist der zusammenfassenden Arbeit von Vögtle und Weber, »Angewandte Chemie«, Band 91 (1979), S. 813 ff, zu entnehmen.

Zweckmäßigerweise verwendet man die Komplexbildner in mindestens stöchiometrischen Mengen zu den Alkali- bzw. Erdalkalimetallkationen. In den meisten Fällen erzielt man die besten Ergebnisse, wenn man pro Grammäquivalent des Kations 1,0—10,0 mol des Komplexbildners einsetzt.

Durch die Komplexbildner werden die Alkali- bzw. Erdalkalialkoholate weitgehend in Lösung gehalten, so daß sich wesentlich geringere Mengen an salzartigen Niederschlägen bilden.

Einen besonderen Vorteil bieten die Komplexbildner ferner bei der destillativen Aufarbeitung des Reaktionsgemisches. Hier kann das Methylformiat vollständig von der methanolischen Katalysatorlösung abdestilliert werden, ohne daß sich in der Destillationsapparatur Ablagerungen und Verkrustungen bilden.

Im übrigen nimmt man die Methylformiatsynthese wie üblich vor, also bei 30—150, vorzugsweise 60—120° C und unter einem Kohlenmonoxiddruck von 50—300, vorzugsweise 200—250 bar.

Als Katalysatoren eignen sich neben den Alkoholaten der Erdalkalimetalle, z. B. Calcium, Strontium oder Barium, besonders die Alkoholate der Alkalimetalle Lithium, Natrium und Kalium, wobei die Verwendung von Natriumalkoholaten in aller Regel am wirtschaftlichsten ist und sich deshalb vor allem empfiehlt. Prinzipiell kann man die Alkoholate beliebiger Alkohole verwenden, jedoch besteht im allgemeinen kein Grund, andere Alkoholate als die des systemeigenen Methanols einzusetzen.

Die Mengen des Katalysators liegen in der Regel zwischen 0,001—0,1, vorzugsweise 0,001—0,01 mol Alkoholat pro Mol Methanol.

## Beispiele 1—9 und Vergleichsbeispiel 1V—3V

Jeweils 100 ml (2,5 mol) Methanol wurden bei 90° C und einem Kohlenmonoxiddruck von p bar im Laufe von t Stunden in Gegenwart von a mol eines Alkalimethylates und b mol eines Komplexbildners in Methylformiat überführt. Die Ergebnisse dieser Versuche hinsichtlich der Ausbeute an Methylformiat, der Farbe des Reaktionsgemisches und der Menge des gebildeten salzartigen Niederschlages sind der tabellarischen Übersicht zu entnehmen.

Beispiele 1–9 und Vergleichsbeispiele 1V–3V, tabellarische Übersicht

| Bsp. | Druck p [bar] | Dauer t [h] | Alkalimet. | a [mol] | Komplexbildner | b [mol] | Farbe Reakt.-gem. | Ausbeute Methylformiat [%] | Niederschlag [g] |
|---|---|---|---|---|---|---|---|---|---|
| 1V | 250 | 3 | Na | 0,006 | – | – | gelb | 98,0 | 1,13 |
| 1 | 250 | 3 | Na | 0,006 | Polyethylenglykolgemisch $HO-(-CH_2-CH_2-O-)_n-H$, n = 3–10 | 0,005 | gelb | 98,0 | 0,5 |
| 2 | 250 | 3 | Na | 0,006 | Polyethylenglykolgemisch $HO-(-CH_2-CH_2-O-)_n-H$, n = 3–10 | 0,01 | gelb | 98,0 | 0,1 |
| 3 | 250 | 3 | Na | 0,006 | Kronenether 1,4,7,10,13-Pentaoxacyclopentadecan (15-Crown-5) | 0,01 | farblos | 96,0 | 0,15 |
| 4 | 250 | 3 | Na | 0,006 | Diethylenglycoldimethylether | 0,015 | bräunlich | 94,2 | 0,44 |
| 2V | 200 | 8 | Li | 0,003 | – | – | gelb | 92,5 | 0,56 |
| 5 | 200 | 8 | Li | 0,003 | 4,7,13,18-Tetraoxa-1,10-diazabicyclo-(8,5,5)-eicosan | 0,002 | bräunlich | 93,0 | 0,24 |
| 6 | 200 | 8 | Li | 0,003 | Tetramethylethylendiamin | 0,015 | gelb | 93,3 | 0,10 |
| 7 | 200 | 8 | Li | 0,003 | Triethanolamin | 0,005 | gelb | 93,6 | 0,11 |
| 3V | 230 | 8 | Na | 0,006 | – | – | gelb | 94,6 | 0,44 |
| 8 | 230 | 8 | Na | 0,006 | 4,7,13,16,21-Pentaoxa-1,10-diazabicyclo-(8,8,5)-tricosan | 0,002 | farblos | 99,0 | 0,10 |
| 9 | 230 | 8 | Na | 0,006 | 1,4,7,10,13-Pentaoxacyclopentadecan | 0,002 | bräunlich | 91,6 | 0,11 |

## Patentansprüche

1. Verfahren zur Herstellung von Methylformiat durch Umsetzung von Methanol und Kohlenmonoxid in Gegenwart eines Alkali- oder Erdalkalialkoholates bei 30—150°C und 50—300 bar, dadurch gekennzeichnet, daß man hierbei einen nichtionischen Komplexbildner zur Bindung der Alkali- bzw. Erdalkalimetallkationen mitverwendet.

2. Verfahren nach Anspruch 1, dadurch gekennzeichnet, daß man als Komplexbildner cyclische oder acyclische Verbindungen mit dem Polyethylenglykol-Strukturelement

$$-(-O-CH_2-CH_2-)_n- \qquad n=2-12$$

verwendet.

3. Verfahren nach den Ansprüchen 1 und 2, dadurch gekennzeichnet, daß man als Alkalialkoholat Na-Methylat verwendet.

## Claims

1. A process for the preparation of methyl formate by reacting methanol and carbon monoxide in the presence of an alkali metal alcoholate or alkaline earth metal alcoholate at from 30 to 150°C and from 50 to 300 bar, wherein a nonionic complexing agent is used to bind the alkali metal cations or alkaline earth metal cations.

2. A process as claimed in claim 1, wherein the complexing agent used is a cyclic or acyclic compound containing the polyethylene glycol structural unit

$$-(-O-CH_2-CH_2-)_n- \qquad n=2-12.$$

3. A process as claimed in claims 1 and 2, wherein the alkali metal alcoholate used is Na methylate.

## Revendications

1. Procédé de préparation du formiate de méthyle par réaction du méthanol et de l'oxyde de carbone entre 30 et 150°C et sous 50 à 300 bars en présence d'un alcoolate de métal alcalin ou alcalino-terreux, caractérisé en ce que l'on emploie en outre un agent complexant non ionique pour fixer les cations du métal alcalin ou alcalinoterreux.

2. Procédé suivant la revendication 1, caractérisé en ce que l'agent complexant est choisi parmi les composés cycliques ou acycliques comprenant un élément de structure polyéthylène-glycol

$$-(-O-CH_2-CH_2-)_n- \qquad \text{avec } n=2 \text{ à } 12.$$

3. Procédé suivant les revendications 1 et 2, caractérisé en ce que l'alcoolate de métal alcalin utilisé est le méthylate de sodium.